(19) Europäisches Patentamt
European Patent Office
Office européen des brevets

(11) Publication number : 0 307 121 B1

(12) # EUROPEAN PATENT SPECIFICATION

(45) Date of publication of patent specification : **18.03.92 Bulletin 92/12**

(51) Int. Cl.$^5$ : **C07D 211/28,** C07D 265/30, A61K 31/445, A61K 31/535

(21) Application number : **88307956.8**

(22) Date of filing : **26.08.88**

(54) **Sulphonamide antiarrhythmic agents.**

(30) Priority : **05.09.87 GB 8720910**

(43) Date of publication of application : **15.03.89 Bulletin 89/11**

(45) Publication of the grant of the patent : **18.03.92 Bulletin 92/12**

(84) Designated Contracting States : **AT BE CH DE ES FR GB GR IT LI LU NL SE**

(56) References cited :
**EP-A- 0 068 669**
**EP-A- 0 140 359**
**EP-A- 0 164 865**
**JOURNAL OF MEDICINAL CHEMISTRY, vol. 10, no. 3, May 1967, pages 462-472; A.A. LARSEN et al.: "Sulfonanilides. II. Analogs of catecholamines"**

(73) Proprietor : **Pfizer Limited**
**Ramsgate Road**
**Sandwich Kent CT13 9NJ (GB)**

(72) Inventor : **Arrowsmith, John Edmund, Dr.**
**340, Dover Road**
**Upper Walmer Deal Kent, CT14 7NX (GB)**
Inventor : **Cross, Peter Edward, Dr.**
**21 Cherry Avenue**
**Canterbury Kent (GB)**

(74) Representative : **Wood, David John et al**
**PFIZER LIMITED, Ramsgate Road**
**Sandwich, Kent CT13 9NJ (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

ANTIARRHYTHMIC AGENTS

This invention relates to certain sulfonamides which are antiarrhythmic agents. EP-A-0 164 865 discloses N-(Aminoalkylphenyl) sulphonamides useful in the treatment of cardiac arrhythmia.

The compounds of the invention prolong the duration of the action potential in cardiac muscle and conducting tissue, and thereby increase refractoriness to premature stimuli. Thus, they are Class III antiarrhythmic agents according to the classification of Vaughan Williams (Anti-Arrhythmic Action, E.M. Vaughan Williams, Academic Press, 1980). They are effective in atria, ventricles and conducting tissue both in vitro and in vivo and are therefore useful for the prevention and treatment of a wide variety of ventricular and supraventicular arrhythmias including atrial and ventricular fibrillation. Because they do not alter the speed at which impulses are conducted, they have less propensity than current drugs (most Class I) to precipitate or aggravate arrhythmias, and also produce less neurological side effects. Some of the compounds also have some positive inotropic activity and therefore are particularly beneficial in patients with impaired cardiac pump function.

Thus the invention provides compounds of the formula:-

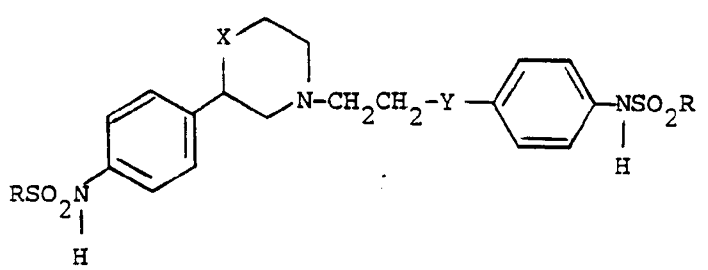

--- (I)

and their pharmaceutically acceptable salts,
where each R, which is the same, is a $C_1$-$C_4$ alkyl group;

X is $CH_2$ or O;

and

Y is O or a direct link.

Each R is preferably $CH_3$.

The pharmaceutically acceptable salts of the compounds of the formula (I) include acid addition salts formed from acids which form non-toxic acid addition salts containing pharmaceutically acceptable anions, such as hydrochloride, hydrobromide, hydroiodide, sulphate or bisulphate, phosphate or hydrogen phosphate, acetate, maleate, fumarate, lactate, tartrate, citrate, gluconate, benzoate, methanesulphonate, besylate and p-toluenesulphonate salts. The compounds also form metal salts, examples of which are the alkaline earth and alkali metal salts, e.g. the sodium and potassium salts. The salts are preparable by conventional techniques.

For assessment of effects of the compounds on atrial refractoriness, guinea pig right hemiatria are mounted in a bath containing physiological salt solution, and one end is connected to a force transducer. Tissues are stimulated at 1 Hz using field electrodes. Effective refractory period (ERP) is measured by introducing premature stimuli ($S_2$) after every 8th basic stimulus ($S_1$). The $S_1S_2$ coupling interval is gradually increased until $S_2$ reproducibly elicits a propagated response. This is defined as the ERP. The concentration of compound required to increase ERP by 25% ($ED_{25}$) is then determined. ERP is also measured in guinea pig right papillary muscles incubated in physiological salt solution. Muscles are stimulated at one end using bipolar electrodes and the propagated electrogram is recorded at the opposite end via a unipolar surface electrode. ERP is determined as above using the extrastimulus technique. Conduction time is obtained from a digital storage oscilloscope by measuring the interval between the stimulus artefact and the peak of the electrogram (i.e. the time required for the impulse to travel along the length of the muscle).

Atrial and ventricular ERP's are also measured in anaesthetised or conscious dogs by the extrastimulus technique whilst the atrium or right ventricle is being paced at a constant rate.

The compounds of the formula (I) can be administered alone but will generally be administered in admixture with a pharmaceutical carrier selected with regard to the intended route of administration and standard pharmaceutical practice. They can be administered both to patients suffering from arrhythmias and also prophylactically to those likely to develope arrhythmias. For example they may be administered orally in the form of tablets containing such excipients as starch or lactose, or in capsules either alone or in admixture with excipients, or

in the form of elixirs or suspensions containing flavouring or colouring agents. They may be injected parenterally, for example, intravenously, intramuscularly or subcutaneously. For parenteral administration, they are best used in the form of a sterile aqueous solution which may contain other solutes, for example, enough salts or glucose to make the solution isotonic.

For administration to man in the curative or prophylactic treatment of cardiac conditions such as ventricular and supraventicular arrhythmias, including atrial and ventricular fibrillation, it is expected that oral dosages of the compounds of the invention will be in the range from 1 to 75 mg daily, taken in up to 4 divided doses per day, for an average adult patient (70 kg). Dosages for intravenous administration would be expected to be within the range 0.5 to 10mg per single dose as required. A severe cardiac arrythmia is preferably treated by the i.v. route in order to effect a rapid conversion to the normal rhythm. Thus for a typical adult patient individual tablets or capsules might for example contain 1 to 25mg of active compound, in a suitable pharmaceutically acceptable vehicle or carrier. Variations may occur depending on the weight and condition of the subject being treated as will be known to medical practitioners.

Thus the present invention provides a pharmaceutical composition comprising a compound of the formula (I) as defined above or pharmaceutically acceptable salt thereof, together with a pharmaceutically acceptable diluent or carrier.

The invention also provides a method of preventing or reducing cardiac arrhythmias in a human being, which comprises administering to said human an effective amount of a compound of the formula (I) or pharmaceutically acceptable salt thereof, or of a pharmaceutical composition as defined above.

The invention yet further provides a compound of the formula (I) or a pharmaceutically acceptable salt thereof, for use as a medicament.

The invention also provides the use of a compound of the formula (I), or of a pharmaceutically acceptable salt thereof, for the manufacture of a medicament for the prevention or reduction of cardiac arrhythmias.

Where the compounds (I) contain an optically active centre, then the invention includes both the separated (R and S) and unseparated forms.

Also included within the scope of the invention are the intermediates of the formula:-

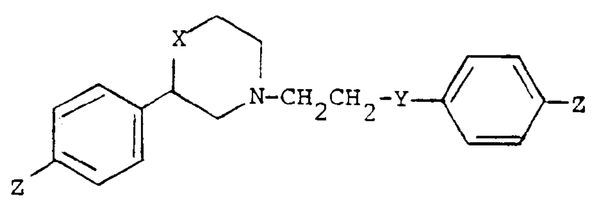

--- (II)

where X and Y are as defined for formula (I), and each Z, which is the same, is $-NO_2$ or $-NH_2$.

The compounds of the formula (I) can be prepared by the acylation of the compounds of the formula (II) in which Z is $-NH_2$ using a $C_1$-$C_4$ alkanesulphonyl chloride or bromide, or a $C_1$-$C_4$ alkanesulphonic anhydride, in a conventional manner.

The reaction is typically carried out in a reaction-inert organic solvent, and preferably in the presence of an acid acceptor. It is most desirable to carry out the reaction in pyridine which functions both as the solvent and the acid acceptor, and to use an alkanesulphonyl chloride or alkanesulphonic anhydride. Generally, the reaction proceeds satisfactorily at temperatures of from 0° to 25°C, i.e., heating is not usually necessary. Preferably, the reaction is carried out at room temperature. The product can be isolated and purified by conventional procedures.

The starting materials of the formula (II) in which X is $CH_2$ can be prepared as follows:-

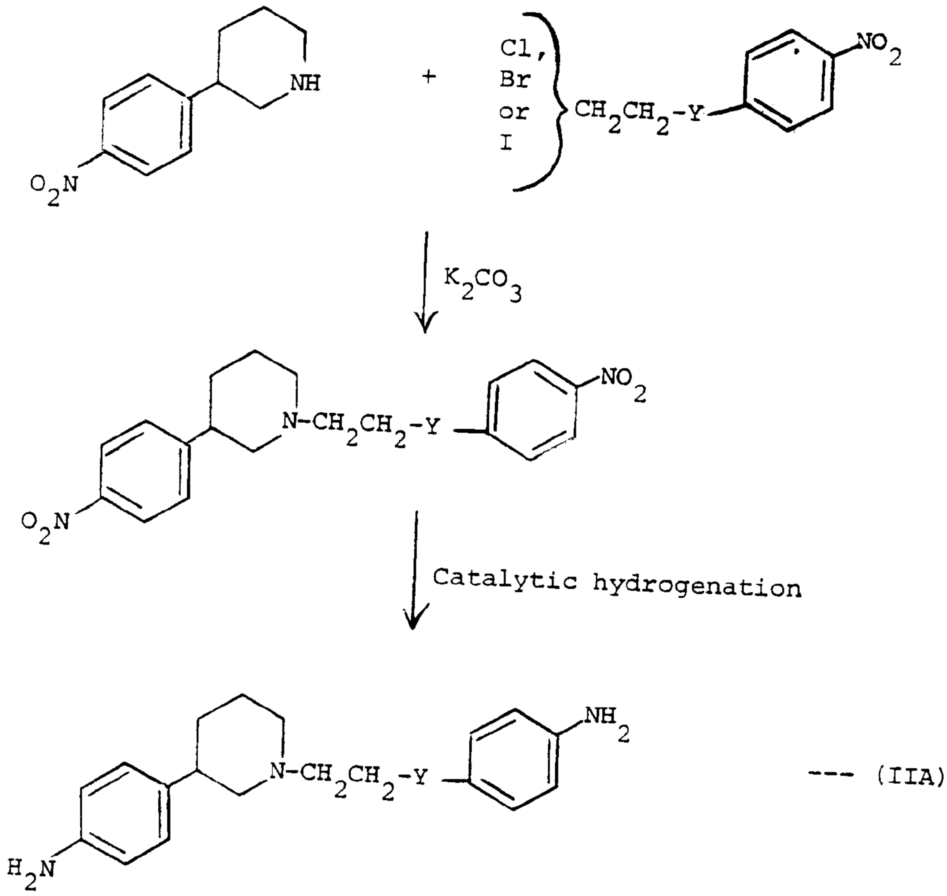


--- (IIA).

The starting materials of the formula (II) in which X is O can be prepared as follows:-

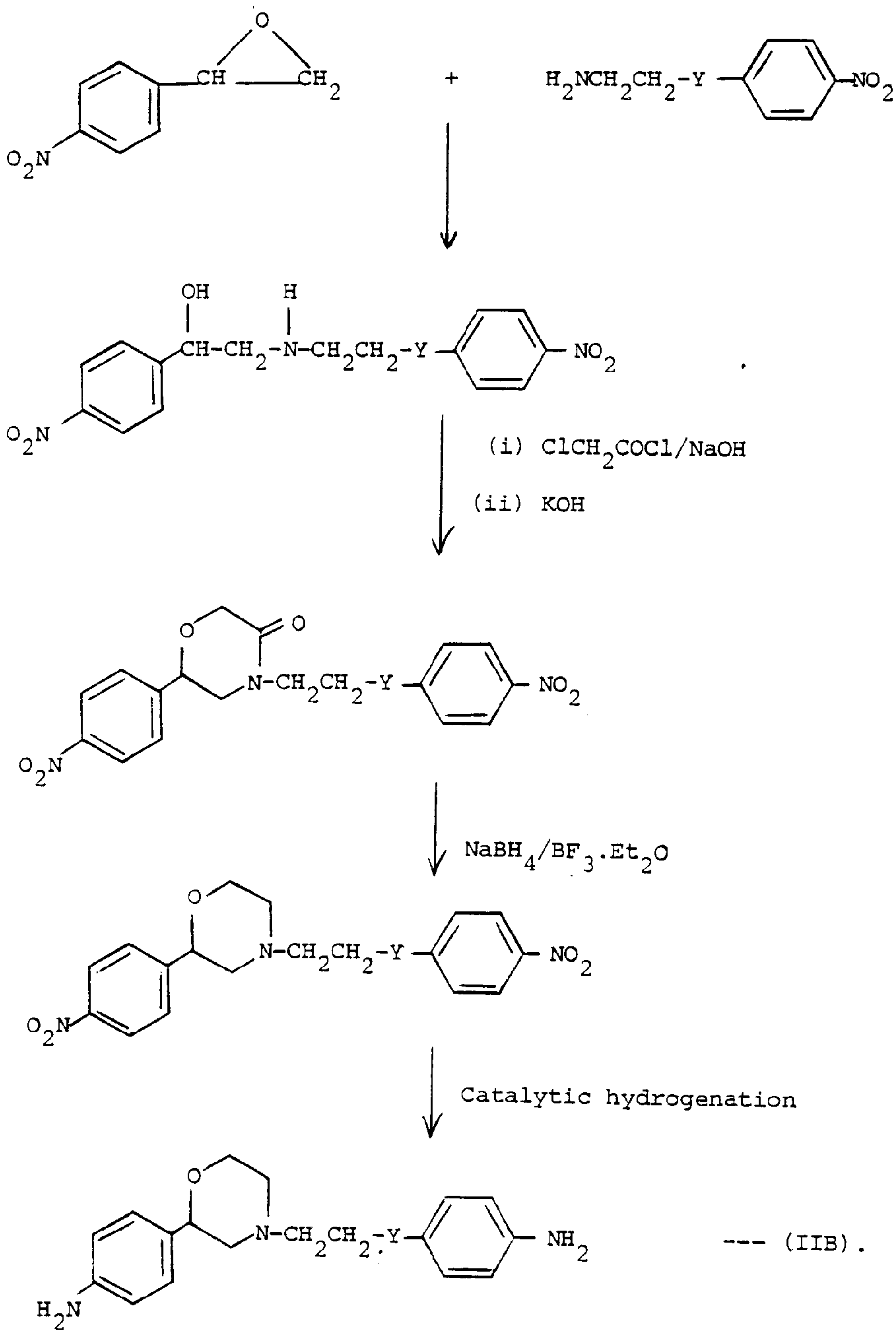


--- (IIB).

The routes to the intermediates (IIA) and (IIB) are described in full detail in the following Preparations.

The following Examples, in which all temperatures are in °C, illustrate the invention:-

EXAMPLE 1

1-[2-(4-Methanesulphonamidophenoxy)ethyl]-3-(4-methanesulphonamidophenyl)piperidine

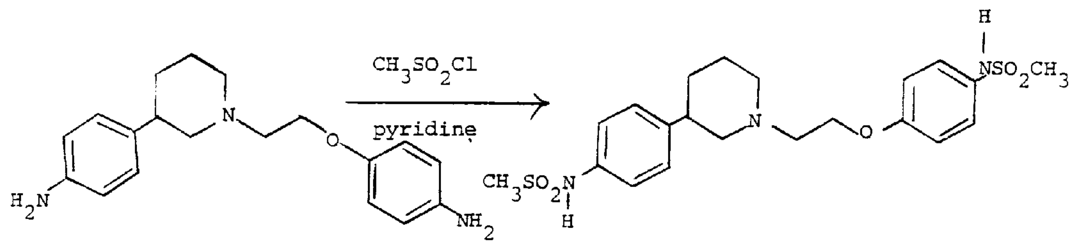


Methanesulphonyl chloride (0.56 ml) was added dropwise to a solution of 1-[2-(4-aminophenoxy)ethyl]-3-(4-aminophenyl)piperidine (1.0 g) in pyridine (50 ml) and the reaction mixture was stirred at room temperature for 17 hours. The solvent was removed by evaporation in vacuo to give a gum which was dissolved in methylene chloride, washed with aqueous sodium bicarbonate, dried ($Na_2SO_4$) and evaporated in vacuo. The residue (1.1 g) still contained some unreacted starting material and so was re-dissolved in methylene chloride containing methanesulphonic anhydride (0.25 g) and stirred for 17 hours at room temperature. The reaction mixture was diluted with aqueous sodium bicarbonate and extracted twice with methylene chloride. The combined organic extracts were dried ($Na_2SO_4$) and evaporated to a residue which was purified by column chromatography on silica eluting with methylene chloride. The product-containing fractions were combined and evaporated to give a colourless solid which was recrystallised from ethyl acetate/petrol to afford the title compound, yield 0.38 g, m.p. 167-169°.

| Analysis %:- | |
|---|---|
| Found: | C,54.0; H,6.3; N,9.1; |
| Calculated for $C_{21}H_{29}N_3O_5S_2$: | C,53.9; H,6.25; N,9.0. |

EXAMPLE 2

1-(4-Methanesulphonamidophenethyl)-3-(4-methanesulphonamidophenyl)piperidine

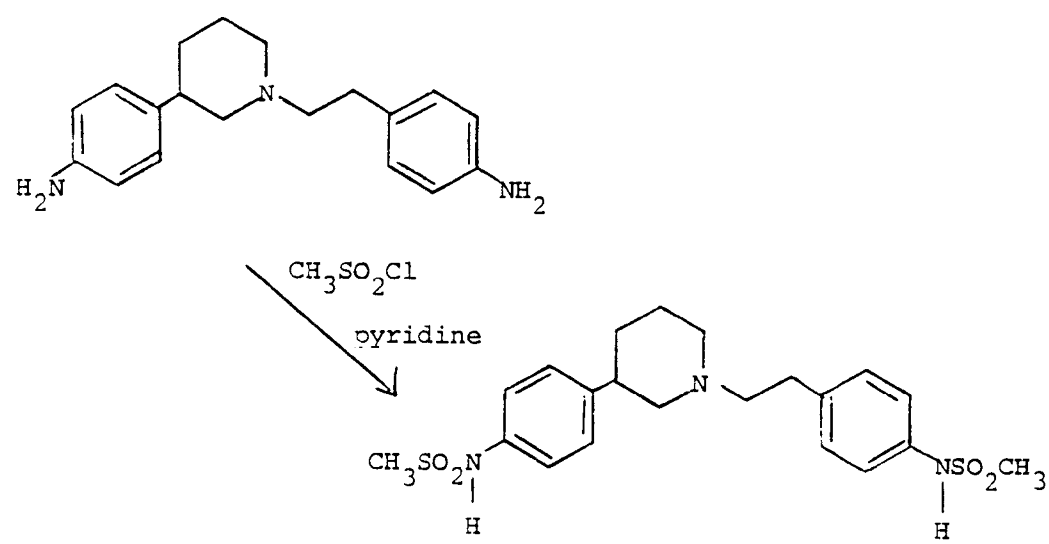


Methanesulphonyl chloride (0.11 ml) was added dropwise to a solution of 1-[4-aminophenethyl]-3-(4-aminophenyl)piperidine (0.2 g) in pyridine (20 ml) and the reaction mixture was stirred at room temperature for 17 hours. The solvent was removed by evaporation in vacuo, the residue taken up in methylene chloride, washed with aqueous sodium bicarbonate, dried ($MgSO_4$) and evaporated in vacuo. The resulting oil was purified by preparative plate chromatography on silica eluting with methylene chloride/methanol (4:1). The product was removed from the silica by washing with methylene chloride/methanol (4:1), followed by filtration and evaporation of the solvent to give the title compound as a foam, yield 0.01 g.

Analysis %:-

| | |
|---|---|
| Found: | C,54.5; H,6.5; N,8.7; |
| Calculated for $C_{21}H_{29}N_3O_4S_2.\frac{1}{2}H_2O$: | C,54.8; H,6.6; N,9.1. |

$^1$H N.m.r. ($CDCl_3$): δ=7.2 (q, 8H); 3.1 (t, 2H); 3.1 (s, 3H); 3.05 (s, 3H); 2.85 (m, 3H); 2.65 (m, 2H); 2.1 (q, 2H); 1.95 (m, 3H); 1.5 (m, 1H).

EXAMPLE 3

4-(4-Methanesulphonamidophenethyl)-2-(4-methanesulphonamidophenyl)morpholine

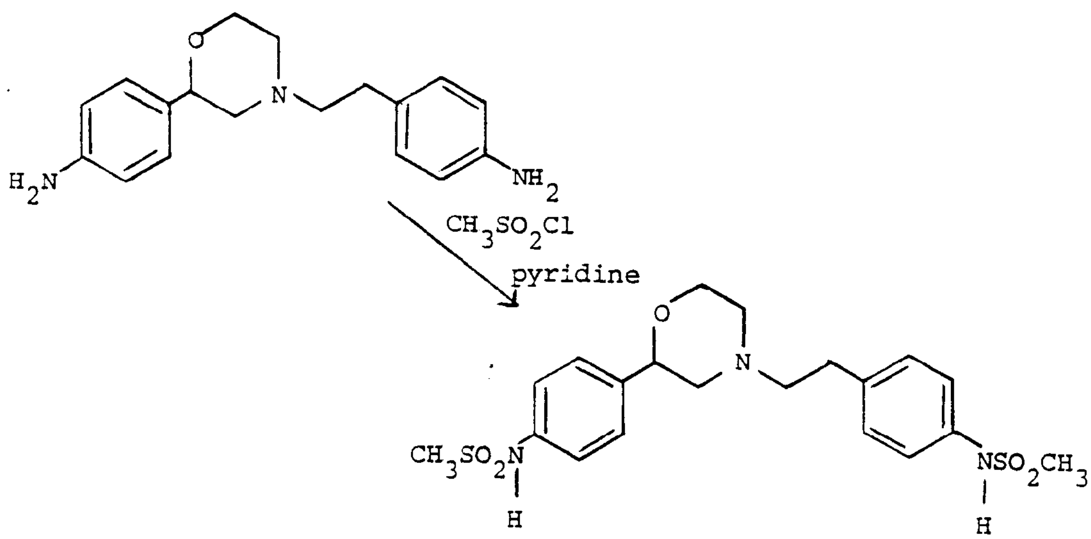


Methanesulphonyl chloride (0.13 ml) was added dropwise to a solution of 4-(4-aminophenethyl)-2-(4-aminophenyl)morpholine (0.25 g) in pyridine and the reaction mixture was stirred at room temperature for 17 hours. The solvent was removed by evaporation in vacuo to give a gum which was purified by column chromatography on silica eluting with methylene chloride containing methanol (0% up to 1%). The product-containing fractions were combined, evaporated in vacuo and the residue was triturated in diisopropyl ether to give the title compound as a colourless precipitate which was collected by filtration and dried in vacuo, yield 0.1 g, m.p. 80-82°.

Analysis %:-

| | |
|---|---|
| Found: | C,53.0; H,6.0; N,9.3; |
| Calculated for $C_{20}H_{27}N_3O_5S_2$: | C,53.3; H,6.3; N,8.9. |

EXAMPLE 4

4-(2-[4-Methanesulphonamidophenoxy]ethyl)-2-(4-methanesulphonamidophenyl)morpholine

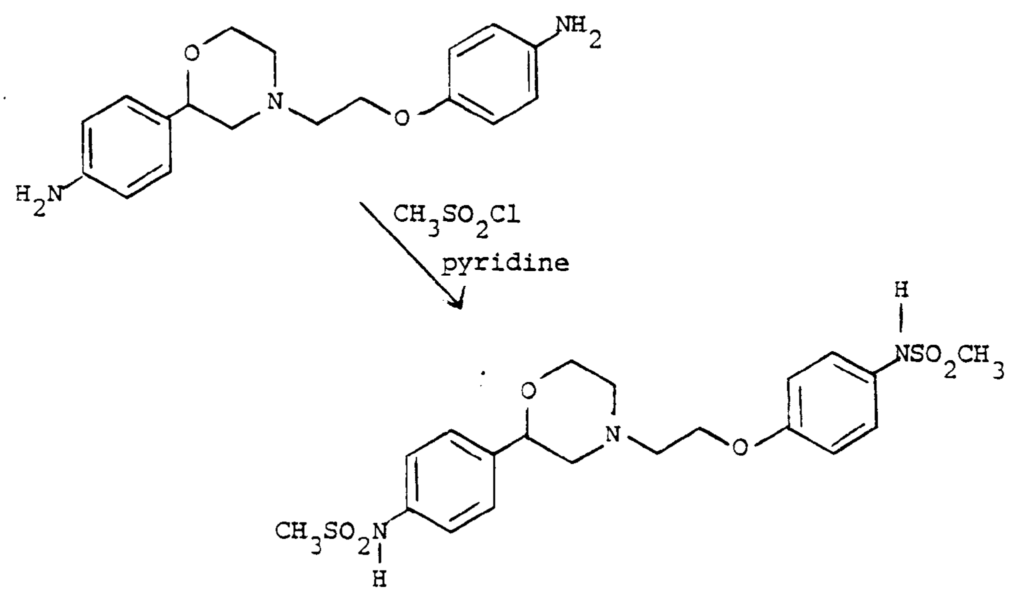


Methanesulphonyl chloride (0.2 ml) was added dropwise to a solution of 4-(2-[4-aminophenoxy]ethyl)-2-(4-aminophenyl)morpholine (0.3 g) in pyridine and the reaction mixture was stirred at room temperature for 23 hours. The solvent was removed by evaporation in vacuo, the residue taken up in methylene chloride, washed (aqueous sodium bicarbonate), dried ($MgSO_4$) and evaporated in vacuo. The resulting gum was dissolved in methylene chloride (3 ml) and the resulting precipitate collected by filtration and recrystallised from ethanol to give the title compound, yield 0.22 g, m.p. 182-184°.

Analysis %:-

| | |
|---|---|
| Found: | C,51.3; H,5.9; N,8.8 |
| Calculated for $C_{20}H_{27}N_3O_6S_2$: | C,51.15; H,5.8; N,8.95. |

The following Preparations, in which all temperatures are in °C, illustrate the preparation of certain starting materials used in the previous Examples:-

Preparation 1

3-(4-Nitrophenyl)piperidine

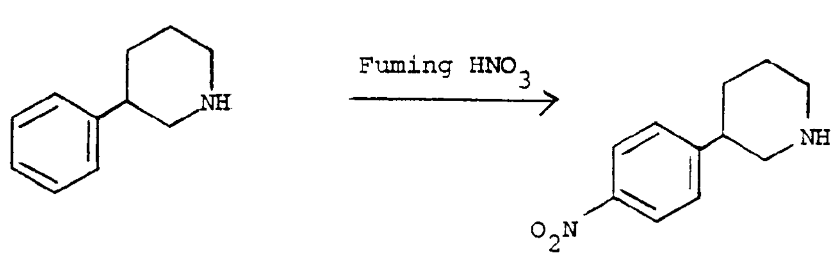


3-Phenylpiperidine (3.0 g) was added dropwise to fuming nitric acid cooled to -10°. Stirring was continued

at this temperature for 1 hour, then the reaction mixture was allowed to warm to room temperature over 1½ hours, and poured into water (100 ml). The aqueous layer was basified with sodium hydroxide (5M) to pH 13-14 and extracted with ether (3 x 100 ml). The combined ether extracts were dried ($MgSO_4$) and evaporated in vacuo to give the title compound as an oil which was used without further purification, yield 2.67 g.

$^1$H N.m.r. ($CDCl_3$) $\delta$ = 8.0 (d, 2H); 7.2 (d, 2H); 2.9 (m, 5H); 1.8 (m, 4H).

The preparation of the title compound is also described in Bull. Soc. Chim. Fr. (1968), 3, 988.

Preparation 2

2-(4-Nitrophenoxy)ethyl chloride

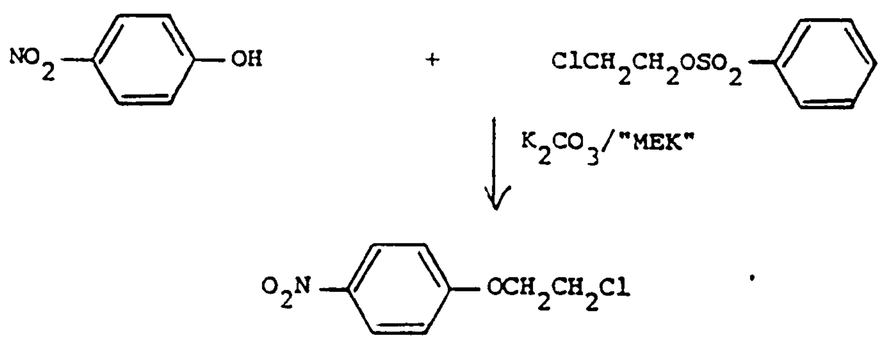


A mixture of 4-nitrophenol (139 g, 1 mole), 2-(benzene-sulphonyloxy)ethyl chloride (220.5 g, 1 mole - see Ber. (1920), 53, 1836) and anhydrous potassium carbonate (138 g, 1 mole) in methyl ethyl ketone ("MEK" - 1000 ml) was stirred at reflux for 16 hours. After cooling, the mixture was poured onto water and the organic layer was separated. Following two further extractions with mehyl ethyl ketone, the combined organic fractions were dried ($MgSO_4$), filtered and evaporated. The resultant solid was crystallised from ethanol to give the title compound, (165.8 g), m.p. 60°.

Analysis %:-

| | |
|---|---|
| Found: | C,47.65; H,4.0; N,7.0; |
| Calculated for $C_8H_8ClNO_3$: | C,47.7; H,4.0; N,7.0. |

Preparation 3

1-[2-(4-Nitrophenoxy)ethyl]-3-(4-nitrophenyl)piperidine

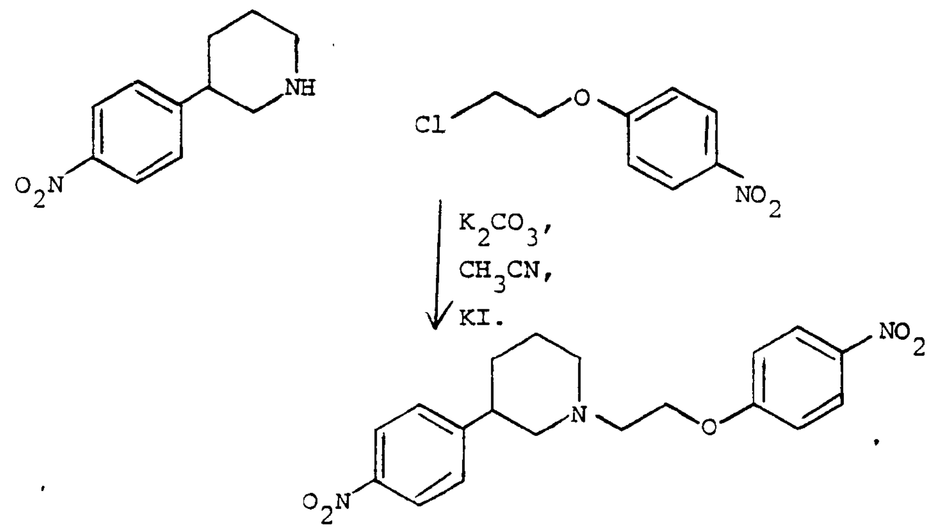


A mixture of 3-(4-nitrophenyl)piperidine (2.06 g), 2-(4-nitrophenoxy)ethyl chloride (1.9 g), potassium carbonate (1.4 g) and sodium iodide (1.5 g) was heated under reflux in acetonitrile (100 ml) for 3 days. The solvent was removed by evaporation and the residue diluted with water (100 ml) and extracted with mehylene chloride (3 x 100 ml). The combined organic extracts were washed (brine), dried ($MgSO_4$) and evaporated. The resulting solid was recrystallised from ethyl acetate to give the title compound, yield 2.0 g, m.p. 130-138°.
$^1$H N.m.r. ($CDCl_3$): δ=8.05 (d, 4H); 7.3 (d, 2H); 6.9 (d, 2H); 4.1 (t, 2H); 2.95 (t, 3H); 2.85 (t, 2H); 2.3 (m, 2H); 1.9 (m, 4H).

Preparation 4

1-[2-(4-Aminophenoxy)ethyl]-3-(4-aminophenyl)piperidine

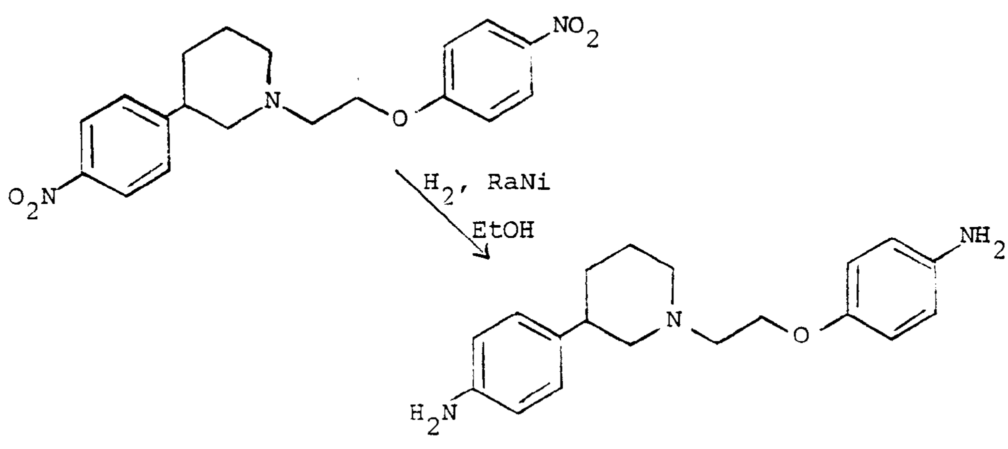


1-[2-(4-Nitrophenoxy)ethyl]-3-(4-nitrophenyl)piperidine (1.75g) in ethanol (100 ml) containing Raney nickel (0.2 g) was stirred under a hydrogen atmosphere [344.6 kPa (50 psi)] for 8 hours at room temperature. The catalyst was removed by filtration and the filtrate was evaporated in vacuo to give the title compound as an oil, yield 1.1 g, which was used directly without further purification.

Preparation 5

1-(4-Nitrophenethyl)-3-(4-nitrophenyl)piperidine

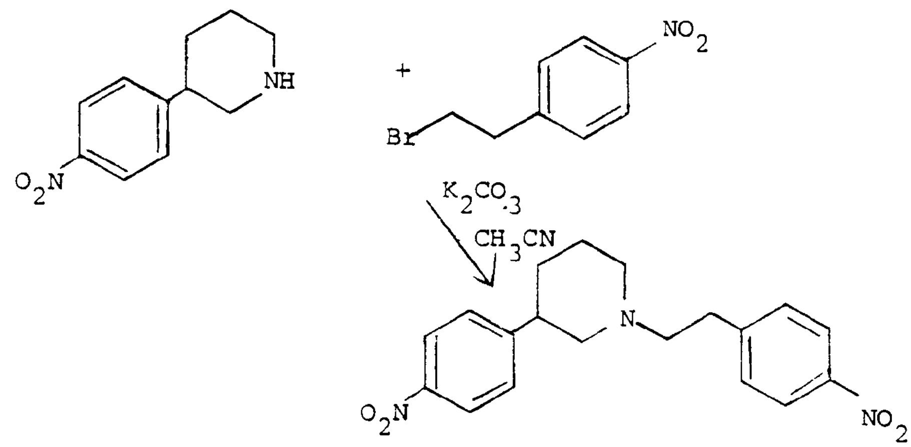


A mixture of 3-(4-nitrophenyl)piperidine (0.42 g), 4-nitrophenethyl bromide (0.47 g) and potassium carbonate (1.0 g) was heated under reflux in acetonitrile (30 ml) for 3 days. The solvent was removed by evaporation and the residue was diluted with water and extracted with ether (3 x 30 ml). The combined organic extracts were dried ($MgSO_4$), evaporated and the residue purified by column chromatography on silica eluting with methylene chloride containing methanol (0% up to 1%). The product-containing fractions were combined and evaporated in vacuo to give a gum which was riturated with diisopropyl ether. The supernatant was evaporated in vacuo to give the title compound, yield 0.5 g.
$^1H$ N.m.r. ($CDCl_3$):δ=8.2 (dd,4H); 7.4 (dd, 4H); 3.1 (d, 2H); 2.95 (t, 3H); 2.7 (t, 2H); 2.1 (q, 2H); 2.0 (broad d, 1H); 1.8 (m, 2H); 1.5 (dq, 1H).

Preparation 6

1-(4-Aminophenethyl)-3-(4-aminophenyl)piperidine

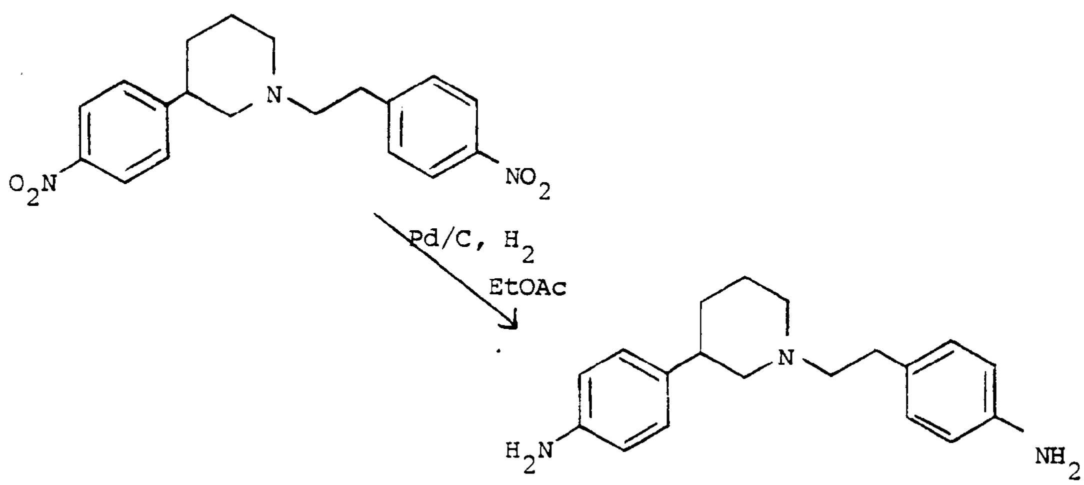


1-(4-Nitrophenethyl)-3-(4-nitrophenyl)piperidine (0.5 g) in ethyl acetate (40 ml) containing 5% Pd/C (0.1 g) was stirred under a hydrogen atmosphere [344.6 kPa (50 psi)] for 3 hours at 30°. The catalyst was removed by filtration and the filtrate was evaporated in vacuo to give the title compound as an oil, yield 0.4 g, which was used directly without further purification.

Preparation 7

N-(4-Nitrophenethyl)-2-(4-nitrophenyl)ethanolamine

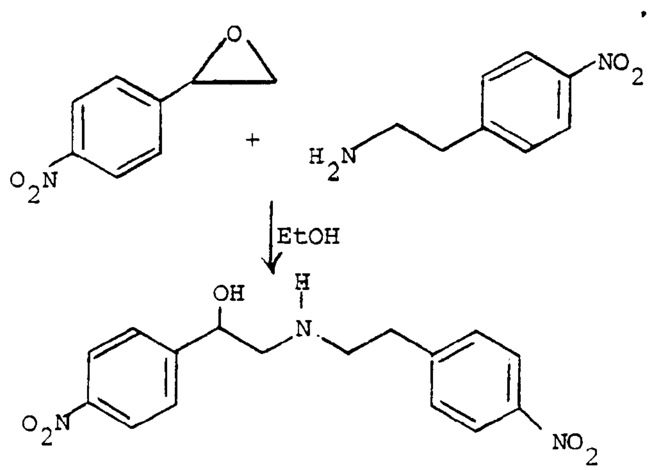


4-Nitrostyrene oxide (3.2 g) (Berichte, 62, 51) and 4-nitrophenethylamine (3.2 g) were stirred for 17 hours in ethanol (75 ml), then heated at reflux for 2$\frac{1}{2}$ hours. The reaction mixture was evaporated to dryness and the residue purified by column chromatography on silica eluting with methylene chloride containing methanol (0% up to 1%). The product-containing fractions were combined and evaporated to give a solid which was recrystallised from ethanol to give the title compound, yield 2.2 g, m.p. 98°.

| | |
|---|---|
| Analysis %:- | |
| Found: | C,58.1: H,5.0; N,12.4; |
| Calculated for $C_{16}H_{17}N_3O_5$: | C,58.0; H,5.2; N,12.7. |

Preparation 8

4-(4-Nitrophenethyl)-2-(4-nitrophenyl)morpholin-5-one

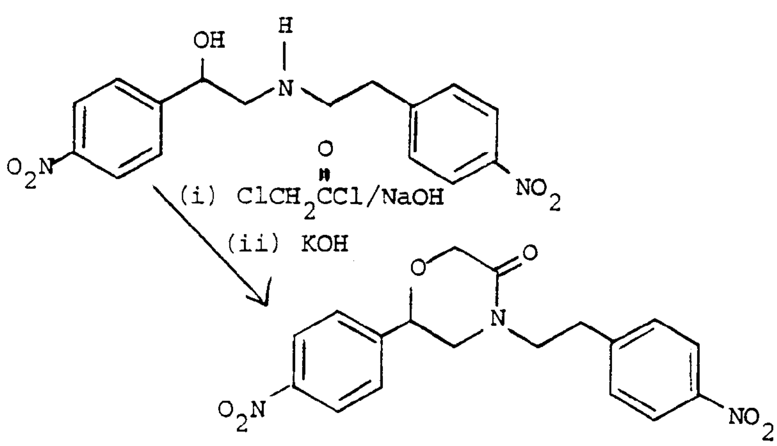


Sodium hydroxide (21 ml, 0.25 M) and then chloroacetyl chloride (0.95 g) in methylene chloride (5 ml) were added dropwise to a vigorously stirred solution of N-(4-nitrophenethyl)-2-(4-nitrophenyl)ethanolamine (2.0 g) in methylene chloride. Stirring was continued for 3$\frac{1}{2}$ hours and then the organic layer was separated, washed (2M hydrochloric acid), dried ($MgSO_4$) and evaporated in vacuo. The resulting foam (2.2 g) was dissolved in ethanol (30 ml) and added portionwise to a solution of potassium hydrozide pellets (0.34 g) in ethanol (10 ml),

and stirring was continued for 3 hours. The mixture was diluted with water (150 ml) and extracted with methylene chloride (4 x 50 ml). The combined organic extracts were dried ($MgSO_4$) and evaporated, giving a solid which was recrystallised from methylene chloride/ethanol to afford the title compound, yield 1.45 g, m.p. 163-164°.

| | |
|---|---|
| Analysis %:- | |
| Found: | C,57.9; H,4.6; N,11.1; |
| Calculated for $C_{18}H_{17}N_3O_6$: | C,58.2; H,4.6; N,11.3. |

Preparation 9

4-(4-Nitrophenethyl)-2-(4-nitrophenyl)morpholine

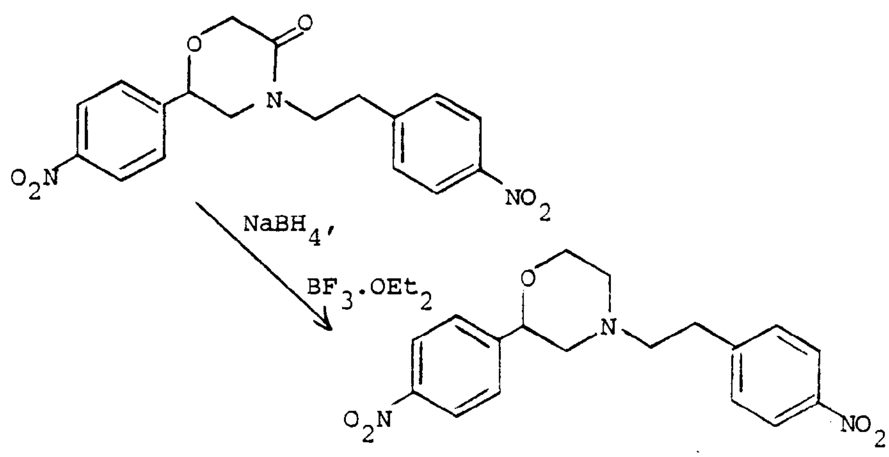


A solution of boron trifluoride diethyl etherate (3.2 ml) in tetrahydrofuran (THF) (20 ml) was added dropwise to a stirred suspension of sodium borohydride (0.72 g) in THF (20 ml) with cooling. The mixture was stirred for a further 5 minutes after the addition was complete and then a solution of 4-(4-nitrophenethyl)-2-(4-nitrophenyl)morpholin-5-one (1.19 g) in THF (40 ml) was added dropwise. The mixture was stirred for 10 minutes, heated at reflux for 1½ hours, cooled, diluted with water (10 ml) and 10% hydrochloric acid (30 ml), and the THF evaporated in vacuo. The aqueous residue was extracted twice with ethyl acetate. The combined organic extracts were washed with aqueous sodium carbonate, dried ($MgSO_4$), evaporated and the residue purified by column chromatography on silica eluting with methylene chloride. The product-containing fractions were combined, evaporated and the residue was crystallised from hot ethanol to give the title compound, yield 0.40 g. m.p. 128°.

| | |
|---|---|
| Analysis %:- | |
| Found: | C,60.6; H,5.4; N,11.8; |
| Calculated for $C_{18}H_{19}N_3O_5$: | C,60.5; H,5.4; N,11.8. |

Preparation 10

4-(4-Aminophenethyl)-2-(4-aminophenyl)morpholine

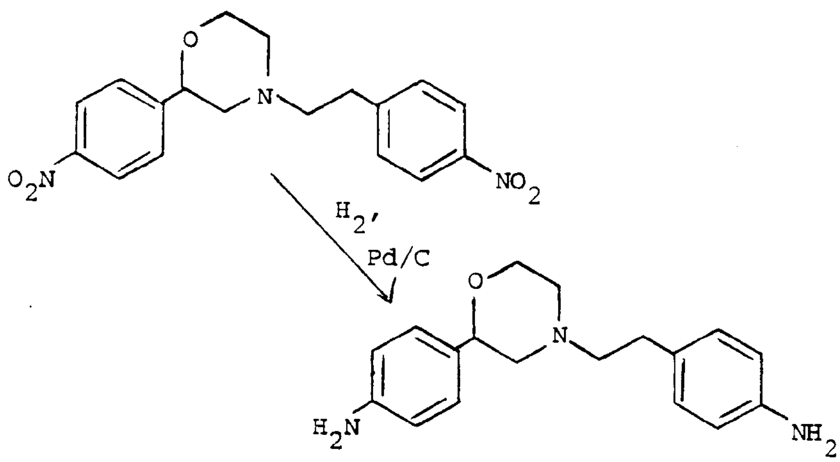


4-(4-Nitrophenethyl)-2-(4-nitrophenyl)morpholine (0.35 g) in ethanol (20 ml) and ethyl acetate (20 ml) containing 5% Pd/C (0.04 g) was stirred under a hydrogen atmosphere [275.7 kPa (40 psi)] for 1 hour at room temperature. The catalyst was removed by filtration and the filtrate evaporated to dryness in vacuo to give the title compound, yield 0.3 g. A sample (0.03 g) was dissolved in ethereal hydrogen chloride to give a colourless precipitate of the trihydrochloride which was filtered, washed, and dried in vacuo, m.p. 200°C (dec).

Analysis %:-

| | |
|---|---|
| Found: | C,51.0; H,6.6; N,9.4; |
| Calcualted for $C_{18}H_{23}N_3O.3HCl.H_2O$: | C,50.9; H,6.6; N,9.8. |

Preparation 11

N-(2-[4-Nitrophenoxy]ethyl)-2-(4-nitrophenyl)ethanolamine

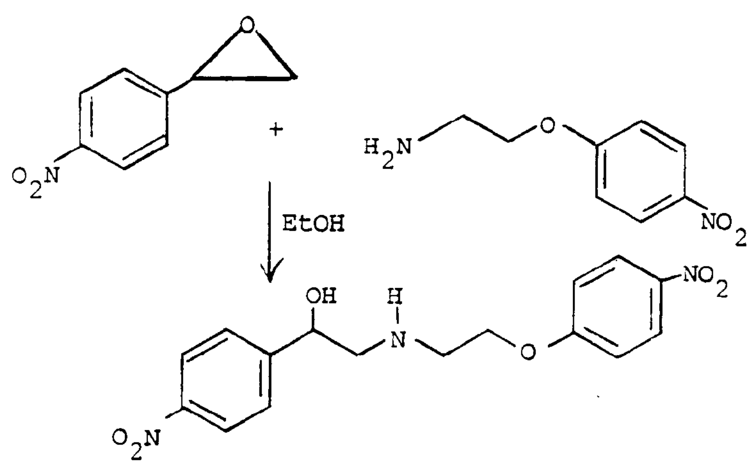


4-Nitrostyrene oxide (2.08 g) and 2-(4-nitrophenoxy)- ethylamine (2.3 g) (Synthesis, 1976, (9), 606) were stirred together for 48 hours in ethanol (100 ml), then heated on a steam bath for 0.5 hours. The volume of ethanol was reduced to 50 ml by evaporation in vacuo and the reaction mixture was stirred at room temperature for 48 hours. The resulting precipitate was collected by filtration, washed with ethanol and then recrystallised

from ethanol to give the title compound, yield 2.32 g, m.p. 145-147°.

Analysis %:-

| | |
|---|---|
| Found: | C,55.3; H,4.9; N,12.1; |
| Calculated for $C_{16}H_{17}N_3O_6$: | C,55.3; H,4.9; N,11.9. |

Preparation 12

4-(2-[4-Nitrophenoxy]ethyl)-2-(4-nitrophenyl)morpholin-5-one

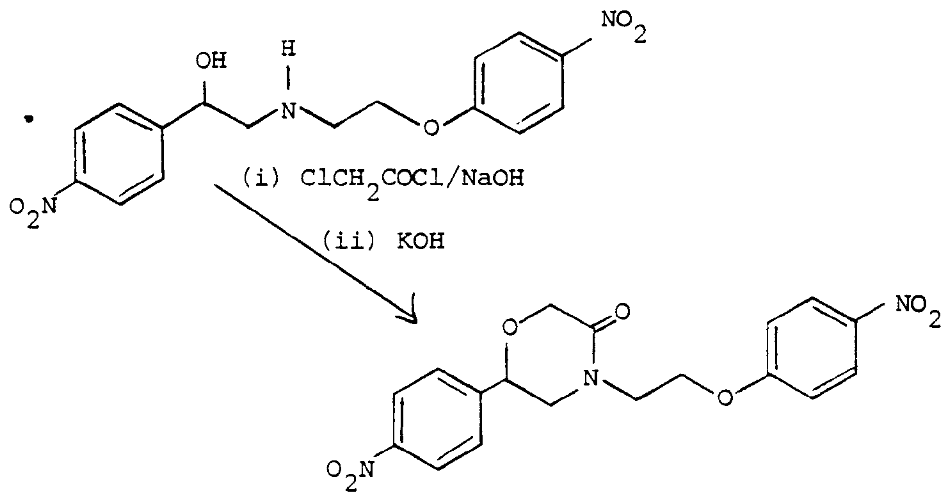


Sodium hydroxide (21 ml, 0.25 M) and then chloroacetyl chloride (0.95 g) in methylene chloride (5 ml) were added dropwise to a vigorously stirred solution of N-(2-[4-nitrophenoxy]ethyl)-2-(4-nitrophenyl)ethanolamine (2.1 g) in methylene chloride (30 ml). Stirring was continued for 3 hours and then the organic layer was separated, washed (2M hydrochloric acid), dried ($MgSO_4$) and evaporated in vacuo. The resulting foam (2.3 g) was dissolved in ethanol (30 ml) and added portionwise to a solution of potassium hydroxide pellets (0.35 g) in ethanol (10 ml), and stirring was continued for 17 hours. The reaction mixture was filtered and the precipitate dissolved in methylene chloride, filtered, and diluted with ethanol. The volume of solvent was then reudced to 5 ml. by evaporation in vacuo to afford the title compound as a precipitate which was collected by filtration and dried, yield 1.25 g, m.p. 187-189°. Addition of more potassium hydroxide (0.2 g) to the original mother liquor afforded a further crop of the product (0.35 g) which was collected by filtration and dried.

Analysis %:-

| | |
|---|---|
| Found: | C,55.7: H,4.4; N,10.8; |
| Calculated for $C_{18}H_{17}N_3O_7$: | C,55.8; H,4.4; N,10.85. |

Preparation 13

4-(2-[4-Nitrophenoxy]ethyl)-2-(4-nitrophenyl)morpholine

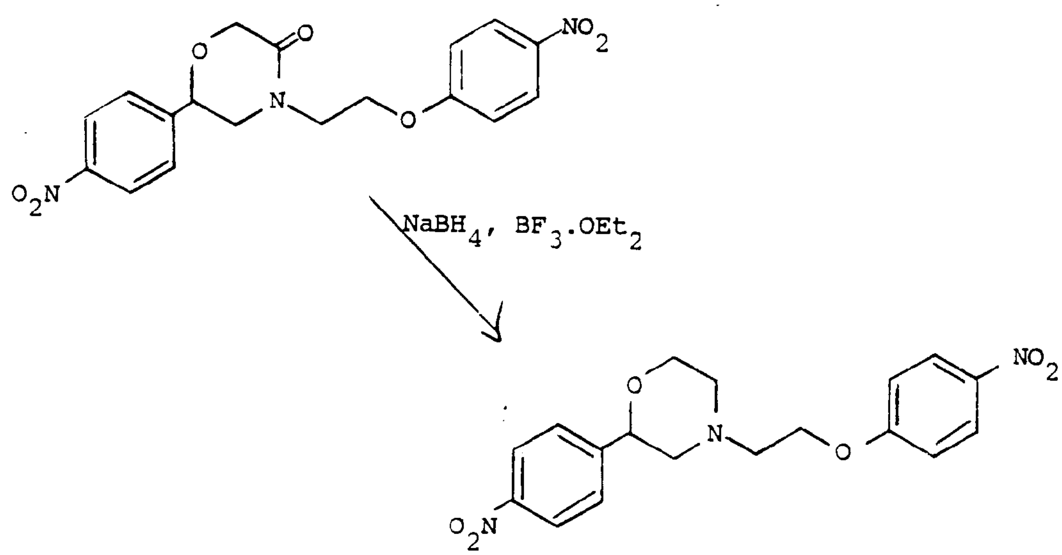


A solution of boron trifluoride diethyl etherate (4.0 ml) in tetrahydrofuran (THF) (20 ml) was added dropwise to a stirred suspension of sodium borohydride (0.9 g) in THF (20 ml) with cooling. The mixture was stirred for a further 5 minutes after the addition was complete and then a solution of 4-(2-[4-nitrophenoxy]ethyl)-2-(4-nitrophenyl)morpholin-5-one (1.55 g) in THF (40 ml) was added dropwise. The mixture was stirred at room temperature for 10 minutes, heated at reflux for $1\frac{1}{2}$ hours, then cooled, dilted with water (10 ml) and 10% hydrochloric acid (35 ml), and the THF evaporated in vacuo. The aqueous residue was extracted twice with ethyl acetate. The combined organic extracts were washed (aqueous sodium carbonate), dried ($MgSO_4$), filtered and evaporated in vacuo to give the title compound, yield 1.5 g. A sample was recrystallised from ethyl acetate, m.p. 166-168°.

Analysis %:-

| | |
|---|---|
| Found: | C,57.9; H,5.1; N,11.3; |
| Calculated for $C_{18}H_{19}N_3O_6$: | C,57.7; H,5.15; N,11.1. |

Preparation 14

4-(2-[4-Aminophenoxy]ethyl)-2-(4-aminophenyl)morpholine

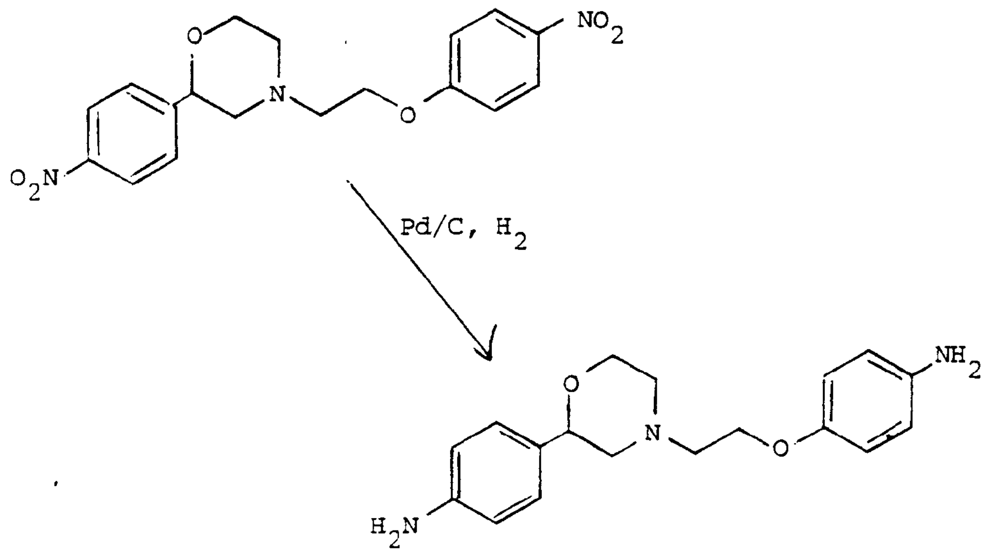


4-2-[4-Nitrophenoxy]ethyl)-2-(4-nitrophenyl)morpholine (0.5 g) in ethyl acetate (50 ml) containing 5% Pd/C (0.05 g) was stirred under a hydrogen atmosphere [275.7 kPa (40 psi)] at 40° for 6 hours. The catalyst was removed by filtration and the filtrate evaporated in vacuo to give the title compound as a gum, yield 0.39 g. A small portion was taken and triturated with ether to give an analytical sample as a powder, m.p. 94-96°.

Analysis %:-

| | |
|---|---|
| Found: | C,68.6; H,7.4; N,13.1; |
| Calculated for $C_{18}H_{23}N_3O_2$: | C,69.0; H,7.4; N,13.4. |

## Claims

**Claims for the following Contracting States: AT, BE, CH, DE, FR, GB, IT, LI, LU, NL, SE**

1. A compound of the formula:

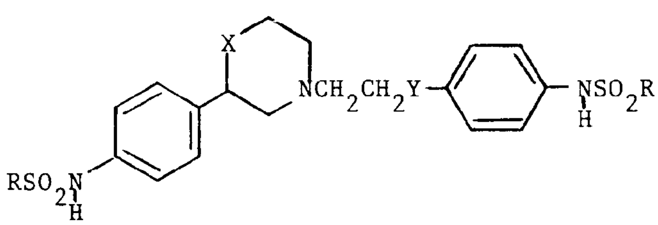
--- (I)

wherein each R, which is the same, is a $C_1$-$C_4$ alkyl group; X is $CH_2$ or O; and Y is O or a direct link; or a pharmaceutically acceptable salt thereof.

2. A compound of the formula (I) as claimed in claim 1 wherein each R is $CH_3$.

3. 1-(4-Methanesulphonamidophenethyl)-3-(4-methanesulphonamidophenyl)piperidine or a pharmaceutically acceptable salt thereof.

4. A compound of the formula:

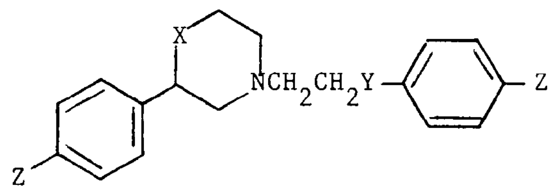

--- (II)

wherein X and Y are as defined in claim 1 and each Z, which is the same, is $-NO_2$ or $-NH_2$.

5. A process for preparing a compound of formula (I) as claimed in claim 1 or a pharmaceutically acceptable salt thereof, which comprises reacting a compound of the formula:

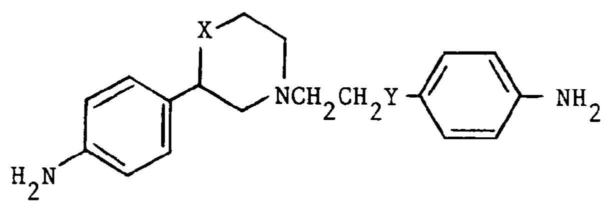

--- (III)

wherein X and Y are as defined in claim 1, with either (a) a $C_1$-$C_4$ alkanesulphonyl chloride or bromide, or (b) a $C_1$-$C_4$ alkanesulphonic anhydride; said process being followed by, optionally, conversion of the product of the formula (I) into a pharmaceutically acceptable salt.

6. A process as claimed in claim 5, wherein the compound of the formula (III) is reacted with a $C_1$-$C_4$ alkanesulphonyl chloride in the presence of an acid acceptor.

7. A pharmaceutical composition comprising a compound of the formula (I) or a pharmaceutically acceptable salt thereof as claimed in any one of claims 1 to 3, together with a pharmaceutically acceptable diluent or carrier.

8. A compound of the formula (I) as claimed in any one of claims 1 to 3, or a pharmaceutically acceptable salt thereof, for use as a medicament.

9. The use of a compound of the formula (I) as claimed in any one of claims 1 to 3, or of a pharmaceutically acceptable salt thereof, for the manufacture of a medicament for the prevention or reduction of cardiac arrhythmias.

**Claims for the following Contracting State: GR**

1. A process for preparing a compound of the formula:

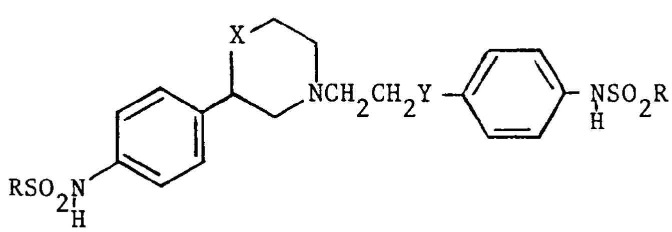

--- (I)

wherein each R, which is the same, is a $C_1$-$C_4$ alkyl group, X is $CH_2$ or O and Y is O or a direct link; or a pharmaceutically acceptable salt thereof, which comprises reacting a compound of the formula:

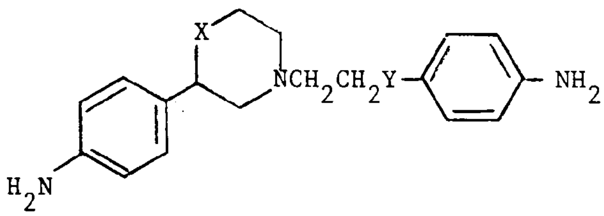

--- (III)

wherein X and Y are as previously defined; with either (a) a $C_1$-$C_4$ alkanesulphonyl chloride or bromide, or (b) $C_1$-$C_4$ alkanesulphonic anhydride; said process being followed by, optionally, conversion of the product of the formula (I) into a pharmaceutically acceptable salt.

2. A process as claimed in claim 1, wherein the compound of the formula (III) is reacted with a $C_1$-$C_4$ alkanesulphonyl chloride in the presence of an acid acceptor.

3. A compound of the formula:

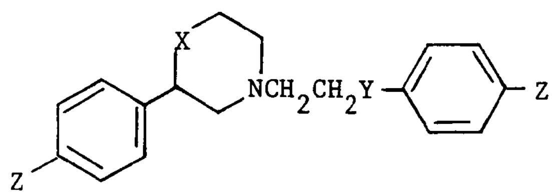

--- (II)

wherein X and Y are as defined in claim 1 and each Z, which is the same, is $-NO_2$ or $-NH_2$.

**Claims for the following Contracting State: ES**

1. A process for preparing a compound of the formula:

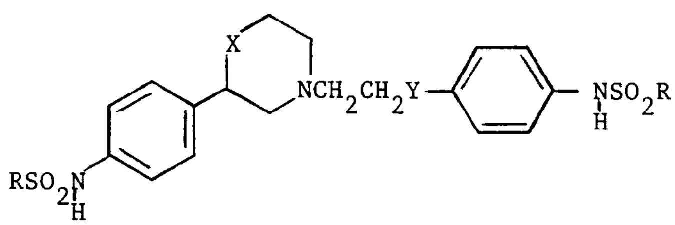

--- (I)

wherein each R, which is the same, is a $C_1$-$C_4$ alkyl group, X is $CH_2$ or O and Y is O or a direct link; or a pharmaceutically acceptable salt thereof, which comprises reacting a compound of the formula:

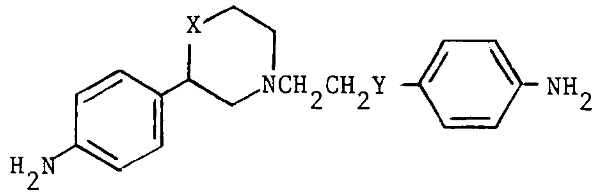

--- (III)

wherein X and Y are as previously defined; with either (a) a $C_1$-$C_4$ alkanesulphonyl chloride or bromide, or (b) a $C_1$-$C_4$ alkanesulphonic anhydride; said process being followed by, optionally, conversion of the product of the formula (I) into a pharmaceutically acceptable salt.

2. A process as claimed in claim 1, wherein the compound of the formula (III) is reacted with a $C_1$-$C_4$ alkanesulphonyl chloride in the presence of an acid acceptor.

## Patentansprüche

### Patentansprüche für folgende Vertragsstaaten: AT, BE, CH, DE, FR, GB, IT, LI, LU, NL, SE

1. Verbindung der Formel

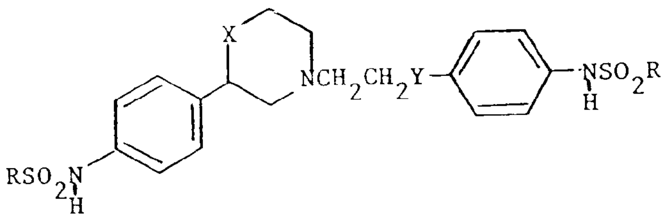

(I),

worin jedes R, das gleich ist, eine $C_1$-$C_4$-Alkylgruppe bedeutet, X $CH_2$ oder O darstellt und Y O oder eine Direktbindung ist, oder oder ein pharmazeutisch annehmbares Salz hievon.

2. Verbindung der Formel (I), wie in Anspruch 1 beansprucht, worin jedes R $CH_3$ ist.

3. 1-(4-Methansulfonamidophenäthyl)-3-(4-methansulfonamidophenyl)-piperidin oder ein pharmazeutisch annehmbares Salz hievon.

4. Verbindung der Formel

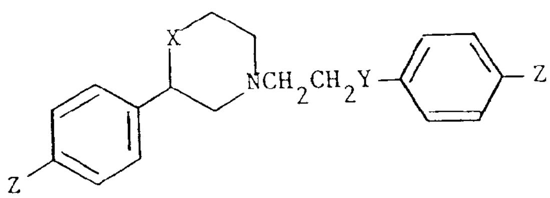

(II),

worin X und Y wie in Anspruch 1 definiert sind und jedes Z, das gleich ist, $-NO_2$ oder $-NH_2$ bedeutet.

5. Verfahren zum Herstellen einer Verbindung der Formel (I), wie in Anspruch 1 beansprucht, oder eines pharmazeutisch annehmbaren Salzes hievon, das das Umsetzen einer Verbindung der Formel

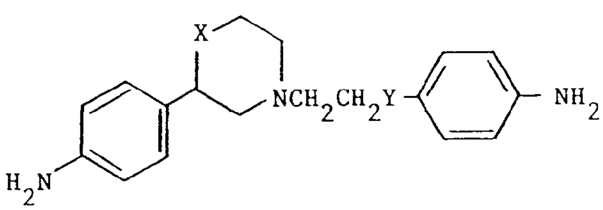

(III),

worin X und Y wie in Anspruch 1 definiert sind, mit entweder (a) einem $C_1$-$C_4$-Alkansulfonylchlorid oder -bromid oder (b) einem $C_1$-$C_4$-Alkansulfonsäureanhydrid umfaßt, auf welches Verfahren gegebenenfalls die Überführung des Produktes der Formel (I) in ein pharmazeutisch annehmbares Salz folgt.

6. Verfahren nach Anspruch 5, in dem die Verbindung der Formel (III) mit einem $C_1$-$C_4$-Alkansulfonylchlorid in Anwesenheit eines Säureakzeptors umgesetzt wird.

7. Pharmazeutische Zusammensetzung umfassend eine Verbindung der Formel (I) oder ein pharmazeutisch annehmbares Salz hievon, wie in einem der Ansprüche 1 bis 3 beansprucht, zusammen mit einem pharmazeutisch annehmbaren Verdünnungsmittel oder Träger.

8. Verbindung der Formel (I), wie in einem der Ansprüche 1 bis 3 beansprucht, oder ein pharmazeutisch annehmbares Salz hievon zur Verwendung als Medikament.

9. Verwendung einer Verbindung der Formel (I), wie in einem der Ansprüche 1 bis 3 beansprucht, oder eines pharmazeutisch annehmbaren Salzes hievon zur Herstellung eines Medikamentes zur Verhütung oder Verminderung von Herzarrhythmien.

**Patentansprüche für folgende Vertragsstaat: GR**

1. Verfahren zum Herstellen einer Verbindung der Formel

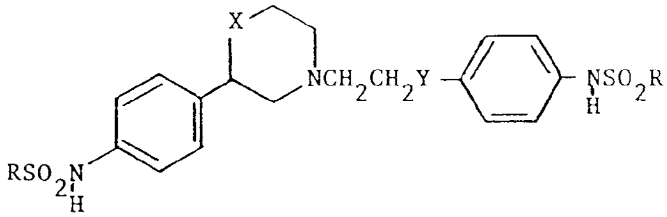

(I),

worin jedes R, das gleich ist, eine $C_1$-$C_4$-Alkylgruppe bedeutet, X $CH_2$ oder O darstellt und Y O oder eine Direktbindung ist, oder eines pharmazeutisch annehmbaren Salzes hievon, das das Umsetzen einer Verbindung der Formel

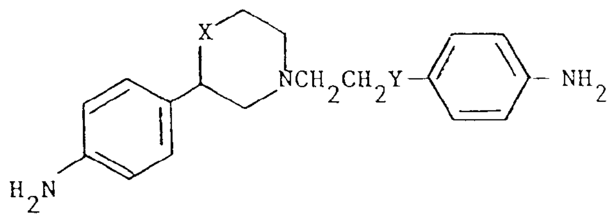

(III),

worin X und Y wie im vorhergehenden definiert sind, mit entweder (a) einem $C_1$-$C_4$-Alkansulfonylchlorid oder -bromid oder (b) einem $C_1$-$C_4$-Alkansulfonsäureanhydrid umfaßt, auf welches Verfahren gegebenenfalls die Überführung des Produktes der Formel (I) in ein pharmazeutisch annehmbares Salz folgt.

2. Verfahren nach Anspruch 1, in dem die Verbindung der Formel (III) mit einem $C_1$-$C_4$-Alkansulfonylchlorid in Anwesenheit eines Säureakzeptors umgesetzt wird.

3. Verbindung der Formel

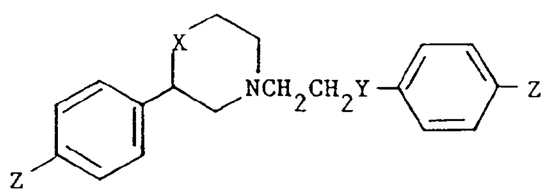

(II),

worin X und Y wie in Anspruch 1 definiert sind und jedes Z, das gleich ist, $-NO_2$ oder $-NH_2$ bedeutet.

**Patentansprüche für folgende Vertragsstaat: ES**

1. Verfahren zum Herstellen einer Verbindung der Formel

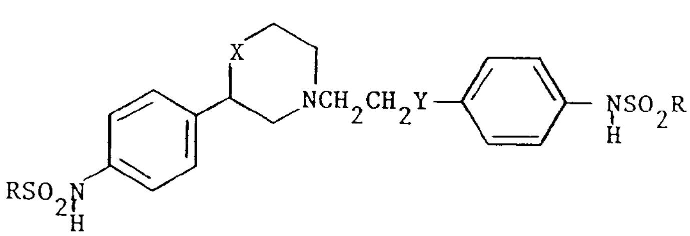

(I),

worin jedes R, das gleich ist, eine $C_1$-$C_4$-Alkylgruppe bedeutet, X $CH_2$ oder O darstellt und Y O oder eine Direktbindung ist, oder eines pharmazeutisch annehmbaren Salzes hievon, das das Umsetzen einer Verbindung der

Formel

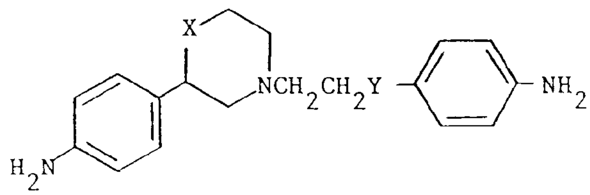

(III),

worin X und Y wie im vorhergehenden definiert sind, mit entweder (a) einem $C_1$-$C_4$-Alkansulfonylchlorid oder -bromid oder (b) einem $C_1$-$C_4$-Alkansulfonsäureanhydrid umfaßt, auf welches Verfahren gegebenenfalls die Überführung des Produktes der Formel (I) in ein pharmazeutisch annehmbares Salz folgt.

2. Verfahren nach Anspruch 1, in dem die Verbindung der Formel (III) mit einem $C_1$-$C_4$-Alkansulfonylchlorid in Anwesenheit eines Säureakzeptors umgesetzt wird.

**Revendications**

**Revendications pour les Etats contractants suivants: AT, BE, CH, DE, FR, GB, IT, LI, LU, NL, SE**

1. Composé de formule :

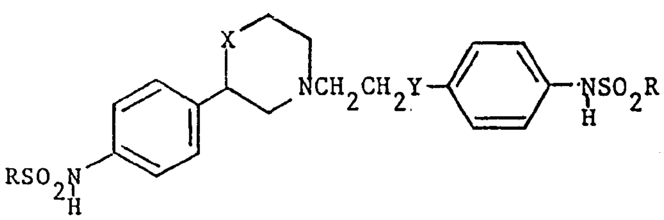

--- (I)

dans laquelle les radicaux R, qui sont identiques, sont des groupes allyle en $C_1$-$C_4$ ; X représente $CH_2$ ou O ; et Y représente O ou une liaison directe ; ou sel pharmaceutiquement acceptable d'un tel composé.

2. Composé de formule (I) selon la revendication 1, dans laquelle chaque radical R représente $CH_3$.

3. 1-(4-méthanesulfonamidophénéthyl)-3-(4-méthanesulfonamidophényl)pipéridine ou sel pharmaceutiquement acceptable de ce composé.

4. Composé de formule :

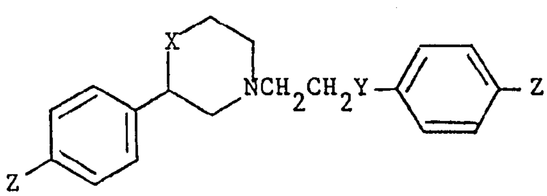

--- (II)

dans laquelle X et Y sont tels que définis dans la revendication 1 et dans laquelle les radicaux Z, qui sont identiques représentent $-NO_2$ ou $-NH_2$.

5. Procédé de préparation d'un composé de formule (I) selon la revendication 1 ou d'un sel pharmaceutiquement acceptable d'un tel composé, qui consiste à faire réagir un composé de formule :

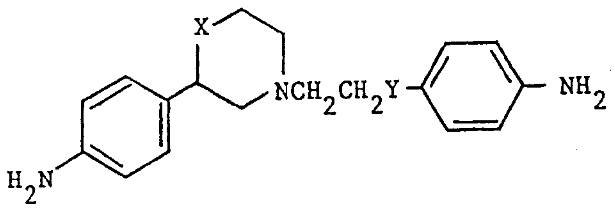

--- (III)

dans laquelle X et Y sont tels que définis dans la revendication 1, soit avec (a) un chlorure ou un bromure d'-(alcane en $C_1$-$C_4$)sulfonyle, ou (b) un anhydride (alcane en $C_1$-$C_4$)sulfonique ; ledit procédé étant suivi, le cas échéant, par la transformation du produit de formule (I) en un sel pharmaceutiquement acceptable.

6. Procédé selon la revendication 5, dans lequel le composé de formule (III) est mis à réagir avec un chlorure d'(alcane en $C_1$-$C_4$)sulfonyle en présence d'un accepteur d'acide.

7. Composition pharmaceutigue renfermant un composé de formule (I), ou un sel pharmaceutiquement acceptable d'un tel composé, selon l'une quelconque des revendications 1 à 3 avec un diluant ou un véhicule pharmaceutiquement acceptables.

8. Composé de formule (I) selon l'une quelconque des revendications 1 à 3, ou sel pharmaceutiquement acceptable d'un tel composé, destiné à être utilisé comme médicament.

9. Utilisation d'un composé de formule (I) selon l'une quelconque des revendications 1 à 3, ou d'un sel pharmaceutiquement acceptable d'un tel composé, pour la fabrication d'un médicament en vue de la prévention ou de la réduction des arythmies cardiaques.

**Revendications pour l'Etat contractant suivant: GR**

1. Préparation d'un composé de formule :

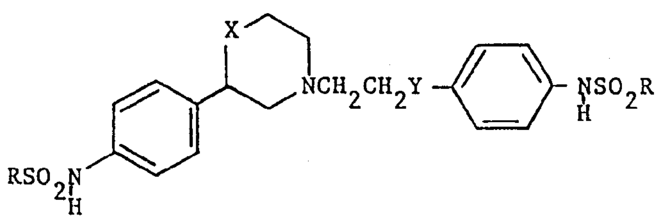

--- (I)

dans laquelle les radicaux R, qui sont identiques, sont des groupes allyle en $C_1$-$C_4$, X représente $CH_2$ ou O et Y représente O ou une liaison directe ; ou d'un sel pharmaceutiquement acceptable d'un tel composé, qui consiste à faire réagir un composé de formule :

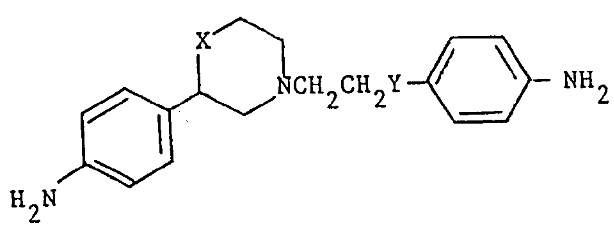

--- (III)

dans laquelle X et Y sont tels que précédemment définis, avec soit (a) un chlorure ou un bromure d'(alcane en $C_1$-$C_4$)sulfonyle, soit (b) un anhydride (alcane en $C_1$-$C_4$)sulfonique ; ledit procédé étant suivi, le cas échéant, par la transformation du composé de formule (I) en un sel pharmaceutiquement acceptable.

2. Procédé selon la revendication 1, dans lequel le composé de formule (III) est mis à réagir avec un chlorure d'(alcane en $C_1$-$C_4$)sulfonyle en présence d'un accepteur d'acide.

3. Composé de formule :

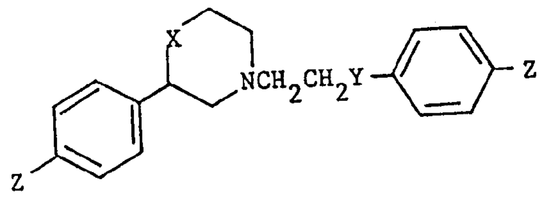

--- (II)

dans laquelle X et Y sont tels que définis dans la revendication 1 et dans laquelle les radicaux Z, qui sont identiques, représentent $-NO_2$ ou $-NH_2$.

**Revendications pour l'Etat contractant suivant: ES**

1. Préparation d'un composé de formule :

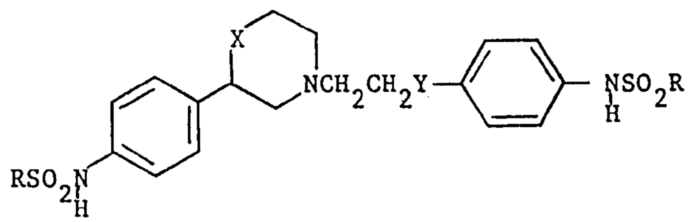

--- (I)

dans laquelle les radicaux R, qui sont identiques, sont des groupes allyle en $C_1$-$C_4$, X représente $CH_2$ ou O et Y représente O ou une liaison directe ; ou d'un sel pharmaceutiquement acceptable d'un tel composé, qui consiste à faire réagir un composé de formule :

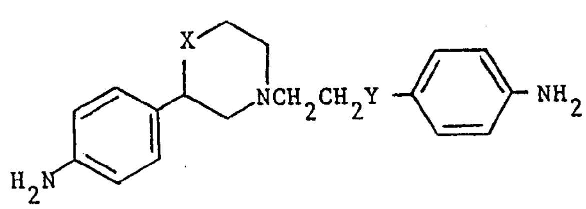

--- (III)

dans laquelle X et Y sont tels que précédemment définis, avec soit (a) un chlorure ou un bromure d'(alcane en $C_1$-$C_4$)sulfonyle, soit (b) un anhydride (alcane en $C_1$-$C_4$)sulfonique ; ledit procédé étant suivi, le cas échéant, par la transformation du composé de formule (I) en un sel pharmaceutiquement acceptable.

2. Procédé selon la revendication 1, dans lequel le composé de formule (III) est mis à réagir avec un chlorure d'(alcane en $C_1$-$C_4$)sulfonyle en présence d'un accepteur d'acide.